# EUROPEAN PATENT APPLICATION

(11) **EP 4 691 564 A2**
(43) Date of publication of application: **11.02.2026**
(21) Application number: 25215036.2
(22) Date of filing: 03.12.2021
(51) Int. Cl.: A61P 35/00

(54) **NEUROKININ INHIBITORS SUCH AS APREPITANT FOR TREATING NON SMALL CELL LUNG CARCINOMA OR BREAST CANCER WITHOUT MUTATIONS**

(30) Priority: 04.12.2020 EP 20383063; 04.06.2021 EP 21382504
(62) Divisional of application: 21820609.2
(71) Applicant: Plus Vitech, S.L., 41510 Mairena del Alcor, Sevilla (ES)
(72) Inventor: MARTIN, Manuel Vicente Salinas, 41013 Sevilla (ES)
(74) Representative: J A Kemp LLP

(57) **Abstract**

The present invention relates to the treatment of ER- / PR- / HER2- breast cancer, using a NK₁ inhibitor.

## Description

### FIELD OF THE INVENTION

The present invention relates to the treatment of non-small cell lung carcinoma (NSCLC) using a NK₁ inhibitor in patients that have wild type EGFR, ALK and ROS1 genes in NSCLC cells. The present invention alternatively or additionally relates to the treatment of breast cancer using a NK₁ inhibitor in patients, in particular breast cancer that does not express estrogen receptors, does not express progesterone receptors, and does not express HER2. The NK₁ inhibitor is preferably aprepitant or fosaprepitant, or pharmaceutically acceptable salt thereof.

### BACKGROUND TO THE INVENTION

Non-small cell lung carcinoma (NSCLC) is any epithelial lung cancer other than small-cell lung carcinoma (SCLC). NSCLC accounts for about 85% of all lung cancers. NSCLCs are relatively insensitive to chemotherapy, compared to SCLC. When possible, they are primarily treated by surgical resection with curative intent, although chemotherapy has been used increasingly both preoperatively (neoadjuvant chemotherapy) and postoperatively (adjuvant chemotherapy).

The most common types of NSCLC are squamous-cell carcinoma, large-cell carcinoma, and adenocarcinoma but several other types occur less frequently. The less common types of NSCLC include pleomorphic, carcinoid tumour, salivary gland carcinoma, and unclassified carcinoma. All types of NSCLC can occur in unusual histologic variants and as mixed cell-type combinations. Lung cancer in people who have never smoked is almost universally NSCLC, with a sizeable majority being adenocarcinoma. Malignant lung tumours may sometimes be found to contain components of both SCLC and NSCLC, in which case the tumours are classified as combined small-cell lung carcinoma (c-SCLC) and are usually treated as "pure" SCLC.

Aprepitant and its prodrug fosaprepitant are neurokinin 1 (NK₁) inhibitors that have been approved for treating nausea and vomiting, for example acute or delayed chemotherapy-induced nausea and vomiting, or post-operative nausea and vomiting. Aprepitant has also been investigated for use in treating a variety of other diseases, including depression. Other NK₁ inhibitors are well known to those skilled in the art.

Despite these therapeutic advances, there remains an urgent need to develop more effective, well-tolerated and affordable treatments to improve symptoms and survival rates for sufferers of NSCLC, and in particular NSCLC having wild type EGFR, ALK and ROS1 genes (EGFR- / ALK- / ROS1-).

Breast cancer is cancer that develops from breast tissue. Signs of breast cancer may include a lump in the breast, a change in breast shape, dimpling of the skin, fluid coming from the nipple, a newly-inverted nipple, or a red or scaly patch of skin. In those with distant spread of the disease, there may be bone pain, swollen lymph nodes, shortness of breath, or yellow skin. Outcomes for breast cancer vary depending on the cancer type, the extent of disease, and the person's age. The five-year survival rates in England and the United States are between 80 and 90%. In developing countries, five-year survival rates are lower. Worldwide, breast cancer is the leading type of cancer in women, accounting for 25% of all cases. In 2018 it resulted in 2 million new cases and 627,000 deaths. It is more common in developed countries and is more than 100 times more common in women than in men.

Breast cancers can be classified by several systems, such as histopathology, grade, stage and receptor status. Classification influences prognosis and can affect treatment response. Description of a breast cancer may include all of these systems. For histopathology, breast cancer is usually classified primarily by its histological appearance. Most breast cancers are derived from the epithelium lining the ducts or lobules, and these cancers are classified as ductal or lobular carcinoma. Carcinoma in situ is growth of low-grade cancerous or precancerous cells within a particular tissue compartment such as the mammary duct without invasion of the surrounding tissue. In contrast, invasive carcinoma does not confine itself to the initial tissue compartment. Grading compares the appearance of the breast cancer cells to the appearance of normal breast tissue. Normal cells in an organ like the breast become differentiated, meaning that they take on specific shapes and forms that reflect their function as part of that organ. Cancerous cells lose that differentiation. In cancer, the cells that would normally line up in an orderly way to make up the milk ducts become disorganised. Cell division becomes uncontrolled. Cell nuclei become less uniform. Cells may be described as well differentiated (low grade), moderately differentiated (intermediate grade), and poorly differentiated (high grade) as the cells progressively lose the features seen in normal breast cells. Poorly differentiated cancers (the ones whose tissue is least like normal breast tissue) have a worse prognosis.

Turning breast cancer staging, the TNM system may be used, which is based on the size of the tumour (T), whether or not the tumour has spread to the lymph nodes (N) in the armpits, and whether the tumour has metastasized (M) (i.e. spread to a more distant part of the body). Larger size, nodal spread, and metastasis have a larger stage number and a worse prognosis. The main stages are: Stage 0 (a pre-cancerous or marker condition, either ductal carcinoma in situ (DCIS) or lobular carcinoma in situ (LCIS)); Stages 1-3 (cancer within the breast or regional lymph nodes); and Stage 4 (metastatic cancer that has a less favourable prognosis since it has spread beyond the breast and regional lymph nodes). Where available, imaging studies may be employed as part of the staging process in select cases to look for signs of metastatic cancer. However, in cases of breast cancer with low risk for metastasis, the risks associated with PET scans, CT scans, or bone scans outweigh the possible benefits, as these procedures expose the person to a substantial amount of potentially dangerous ionising radiation.

Assessment of receptor status makes use of the presence or absence of certain receptors associated with breast cancer cells. These receptors may be present on the surface, cytoplasm and/or nucleus of a breast cancer cells. Chemical messengers such as hormones bind to receptors, and this causes changes in the cell. Breast cancer cells may or may not have three important receptors: estrogen receptor (ER), progesterone receptor (PR), and HER2. ER+ cancer cells (that is, cancer cells that express an estrogen receptor) depend on estrogen for their growth, so they can be treated with drugs to block estrogen effects (e.g. tamoxifen), and generally have a better prognosis. HER2+ breast cancer cells respond to drugs such as the monoclonal antibody trastuzumab (which may be used in combination with conventional chemotherapy). Cells that do not express any of these three receptor types (an estrogen receptor, progesterone receptor, or HER2) are called triple negative, although they may express receptors for other hormones, such as androgen receptor and prolactin receptor.

The management of breast cancer depends on various factors, including the stage of the cancer and age. Treatments are more aggressive when the breast cancer is more advanced or there is a higher risk of recurrence of the breast cancer following treatment. Breast cancer is usually treated with surgery, which may be followed by chemotherapy, radiation therapy, hormonal therapy, chemotherapy, antibody therapy, or any combination thereof and a multidisciplinary approach may be preferable. Hormone receptor-positive cancers are often treated with hormone-blocking therapy over courses of several years. Monoclonal antibodies, or other immune-modulating treatments, may be administered in certain cases of metastatic and other advanced stages of breast cancer.

Some breast cancers require estrogen to continue growing. They can be identified by the presence of an estrogen receptor (ER+) and a progesterone receptor (PR+) on their surface (sometimes referred to together as hormone receptors). These ER+ breast cancers can be treated with hormone therapy, for example using drugs that either block the receptors, e.g. tamoxifen, or alternatively block the production of estrogen with an aromatase inhibitor, e.g. anastrozole or letrozole. The use of tamoxifen is recommended for 10 years. Letrozole is recommended for 5 years. Aromatase inhibitors are only suitable for women after menopause; however, in this group, they appear better than tamoxifen. This is because the active aromatase in postmenopausal women is different from the prevalent form in premenopausal women, and therefore these agents are ineffective in inhibiting the predominant aromatase of premenopausal women. Aromatase inhibitors should not in general be given to premenopausal women with intact ovarian function (unless they are also on treatment to stop their ovaries from working). CDK inhibitors can be used in combination with endocrine or aromatase therapy.

Chemotherapy is predominantly used for cases of breast cancer in stages 2-4, and is particularly beneficial in estrogen receptor-negative (ER-) disease. The chemotherapy medications are administered in combinations, usually for periods of 3-6 months. One of the most common regimens, known as "AC", combines cyclophosphamide with doxorubicin. Sometimes a taxane drug, such as docetaxel, is added, and the regime is then known as "CAT". Another common treatment is cyclophosphamide, methotrexate, and fluorouracil (or "CMF"). Most chemotherapy medications work by destroying fast-growing and/or fast-replicating cancer cells, either by causing DNA damage upon replication or by other mechanisms. However, the medications also damage fast-growing normal cells, which may cause serious side effects. Damage to the heart muscle is the most dangerous complication of doxorubicin, for example.

Antibodies, and in particular monoclonal antibodies, can be used to treat certain types of breast cancer. For example, trastuzumab, a monoclonal antibody to HER2, has improved the 5-year disease free survival of stage 1-3 HER2-positive breast cancers to about 87% (overall survival 95%). Between 25% and 30% of breast cancers overexpress the HER2 gene or its protein product, and overexpression of HER2 in breast cancer is associated with increased disease recurrence and worse prognosis. Trastuzumab, however, is very expensive, and its use may cause serious side effects (approximately 2% of people who receive it develop significant heart damage). Another antibody pertuzumab prevents HER2 dimerization and may be used together with trastuzumab and chemotherapy in severe disease.

Aprepitant and its prodrug fosaprepitant are neurokinin 1 (NK₁) inhibitors that have been approved for treating nausea and vomiting, for example acute or delayed chemotherapy-induced nausea and vomiting, or post-operative nausea and vomiting. Aprepitant has also been investigated for use in treating a variety of other diseases, including depression. Other NK₁ inhibitors are well known to those skilled in the art.

Despite these therapeutic advances, there remains an urgent need to develop more effective, well-tolerated and affordable treatments to improve symptoms and survival rates for sufferers of breast cancer, and in particular breast cancer not expressing, or substantially not expressing, all three of estrogen receptor (ER), progesterone receptor (PR), and HER2 (i.e. "triple negative" breast cancer with a ER- / PR- / HER2- molecular profile). These triple negative breast cancers are not amenable to treatment with specific hormone and/or antibody therapies discussed above. Triple negative breast cancers account for about 10-15% of all breast cancers and tend to grow and spread faster, have limited treatment options, and a worse prognosis compared to other types of breast cancer.

### SUMMARY OF THE INVENTION

It has now been found that the specific subgroup of NSCLC with the EGFR- / ALK- / ROS 1- profile has a surprisingly improved response to treatment with a NK₁ inhibitor (such as aprepitant or fosaprepitant, or a pharmaceutically acceptable salt thereof). The subgroup of patients with NSCLC with an EGFR- / ALK- / ROS1-molecular profile constitutes a subgroup of patients with an especially poor prognosis compared to patients whose tumours present a molecular profile such as EGFR+ / ALK- / ROS1- or EGFR- / ALK+ / ROS 1-. This is because patients whose lung cancers present an EGFR+ / ALK- / ROS 1- molecular profile are candidates for treatment with drugs directed against specific EGFR therapeutic targets, such as Erlotinib or Gefitinib that can provide an improvement in overall survival and in progression-free survival. Likewise, patients whose lung cancers present an EGFR- / ALK+ / ROS1- or ROS1+ molecular profile are candidates for treatment with drugs directed against the specific EGFR therapeutic targets, such as Crizotinib or Ceritinib, which likewise provide an improvement in survival in these patients.

Patients whose lung cancers present an EGFR- / ALK- / ROS1- profile are not candidates for treatment with these targeted treatments and would only be candidates for immunotherapy treatment if they present high PD1-PDL1 expression. It is, however, in these patients that a striking improvement in the response to treatment with a NK₁ inhibitor was observed and therefore these patents represent a promising subset of patient for treating with a NK₁ inhibitor, providing an effective therapeutic tool for NSCLC patient who are not candidates for or have become resistant to immunotherapy treatment and therefore representing a subset of NSCLC patients with an especially poor prognosis. Further, NK₁ inhibitors are safe at doses up to at least 100 mg/kg/day and no macroscopic or microscopic signs of toxicity are seen at these doses. Thus, the enhanced therapeutic effect that is observed with the use of a NK₁ inhibitor in NSCLC having the molecular profile EGFR- / ALK- / ROS 1- is highly advantageous from a clinical perspective.

It has also now been found that the specific subgroup of breast cancer with the triple negative ER- / PR- / HER2- molecular profile has a surprisingly improved response to treatment with a NK₁ inhibitor (such as aprepitant or fosaprepitant, or a pharmaceutically acceptable salt thereof). The subgroup of patients with breast cancer with the triple negative ER- / PR- / HER2- molecular profile constitutes a subgroup of patients whose breast cancer is not amenable to treatment with drugs, or combinations of drugs, targeting an ER, a PR and/or HER2. This is in contrast to patients whose tumours present a molecular profile such as ER- / PR- / HER2+, ER+ / PR- / HER2-, ER- / PR+ / HER2-, ER+ / PR+ / HER2-, ER- / PR+ / HER2+ and ER+ / PR+ / HER2+, who could be treated with such drugs.

It is, however, in these triple negative patients that a striking improvement in the response to treatment with a NK₁ inhibitor was observed and therefore these patents represent a promising subset of patient for treating with a NK₁ inhibitor, providing an effective therapeutic tool for treating triple negative breast cancer patients. Further, NK₁ inhibitors are safe at doses up to at least 100 mg/kg/day and no macroscopic or microscopic signs of toxicity are seen at these doses. Thus, the enhanced therapeutic effect that is observed with the use of a NK₁ inhibitor in breast cancers having the a triple negative ER- / PR- / HER2- molecular profile is highly advantageous from a clinical perspective.

The present invention provides in a first embodiment a pharmaceutical composition which comprises a NK₁ inhibitor, for use in treating: non-small cell lung carcinoma (NSCLC) wherein at least 1% of the cells in a NSCLC tumour to be treated have wild type EGFR, ALK and ROS1 genes; and/or breast cancer wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry.

The present invention provides in a second embodiment a NK₁ inhibitor for use in treating: NSCLC, wherein at least 1% of the cells in a NSCLC tumour to be treated have wild type EGFR, ALK and ROS1 genes; and/or breast cancer, wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry.

The present invention provides in a third embodiment a method of treating a patient: suffering from NSCLC wherein at least 1% of the cells in a NSCLC tumour to be treated have wild type EGFR, ALK and ROS1 genes, which method comprises administering to said patient a NK₁ inhibitor; and/or suffering from breast cancer wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry, which method comprises administering to said patient a NK₁ inhibitor.

The present invention provides in a fourth embodiment the use of a NK₁ inhibitor in the manufacture of a medicament for the treatment of: NSCLC, wherein at least 1% of the cells in a NSCLC tumour to be treated have wild type EGFR, ALK and ROS1 genes; and/or breast cancer, wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry.

In the first, second, third and/or fourth embodiment defined above, the NK₁ inhibitor may be selected from aprepitant, fosaprepitant, netupitant, maropitant, vestipitant, casopitant, vofopitant, ezlopitant or lanepitant, or a pharmaceutically acceptable salt thereof such as fosaprepitant dimeglumine.

### BRIEF DESCRIPTION OF THE FIGURES

Figure 1 shows a graphical representation of the body weight mean, on the "Y" axis, in relation to the day of evolution, on the "X" axis, for each dose of aprepitant administered (mg/kg/day). Data based upon Table 1.
Figure 2 shows a graphical representation of the proliferation mean, on the "Y" axis in relation to the dose of aprepitant administered (µM). Data based upon Table 4.
Figure 3 shows a graphical representation of the proliferation mean, on the "Y" axis in relation to the dose of aprepitant administered (µM) broken down by each cell line with EGFR- / ALK- / ROS1- molecular profile. "Line 1" and "Line 2" show the dose cut-offs corresponding respectively to 5 µM (~625 mg daily) and 20 µM (~1000 mg daily) of aprepitant. Data based upon Tables 5 and 6.
Figure 4 shows a graphical representation of the proliferation mean and standard deviation (SD) of cultured primary invasive breast carcinoma cells with the molecular profile ER- / PR- / HER2- in relation to the dose of aprepitant administered to the primary cells (µM). Data based upon Table 7.
Figure 5 shows a graphical representation of the proliferation mean and standard deviation (SD) of cultured breast cancer cell lines with the indicated molecular profile in relation to the dose of aprepitant administered (µM). 5 µM corresponds to a dose of ~625 mg daily and 20 µM corresponds to a dose of ~1000 mg daily of aprepitant. Data based upon Table 9.

### DETAILED DESCRIPTION

### NK₁ inhibitors

NK₁ inhibitors are a well-known class of drug, and any suitable NK₁ inhibitor can be used in the present invention. NK₁ inhibitors may also be referred to as NK₁ antagonists, NK₁ receptor antagonists or NK₁ receptor inhibitors.

Typically, the NK₁ inhibitor is aprepitant, fosaprepitant, netupitant, maropitant, vestipitant, casopitant, vofopitant, ezlopitant, lanepitant, LY-686017, L-733,060, L-732,138, L -703,606, WIN 62,577, CP-122721, TAK-637, R673, CP-100263, WIN 51708, CP-96345, L-760 735, CP-122721, L-758 298, L-741 671, L-742 694, CP-99994 or T-2328, or a pharmaceutically acceptable salt of any thereof.

Preferably, the NK₁ inhibitor is aprepitant, fosaprepitant, netupitant, maropitant, vestipitant, casopitant, vofopitant, ezlopitant or lanepitant, or a pharmaceutically acceptable salt of any thereof.

More preferably, the NK1 inhibitor is aprepitant, fosaprepitant or netupitant, maropitant, or a pharmaceutically acceptable salt of any thereof.

Most preferably, the NK1 inhibitor is aprepitant or its prodrug fosaprepitant, or a pharmaceutically acceptable salt of either thereof.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as citric, fumaric, maleic, malic, ascorbic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases such as alkyl amines such as meglumine, aralkyl amines or heterocyclic amines.

Aprepitant has the following structure: 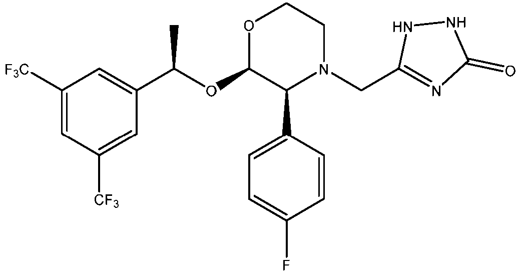 Aprepitant is not typically formulated in the form of a pharmaceutically acceptable salt. Thus, in a preferred aspect of the invention the NK₁ inhibitor is aprepitant.

Fosaprepitant is prodrug of aprepitant and has the following structure:

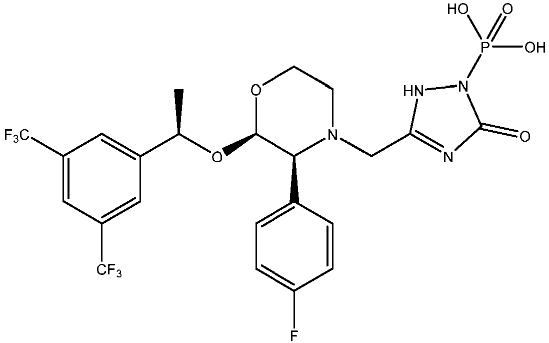

Fosaprepitant is typically provided in the form of a pharmaceutically acceptable salt, preferably in the form of the dimeglumine salt:

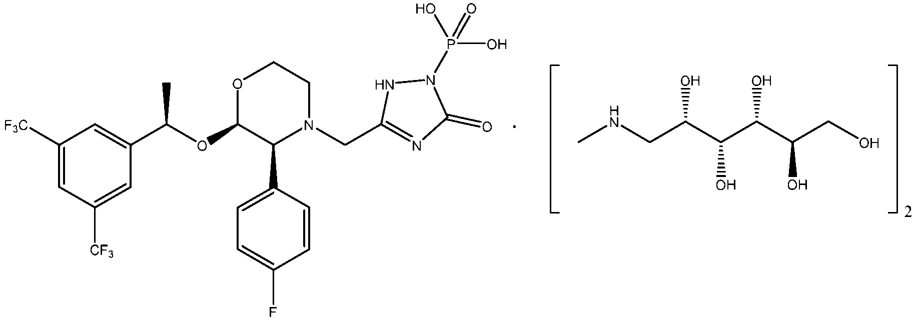

Thus, in a preferred aspect of the invention, the NK₁ inhibitor is fosaprepitant dimeglumine.

Pharmaceutically acceptable salts of fosaprepitant, such as fosaprepitant dimeglumine, are typically reconstituted in an aqueous solvent, such as saline, prior to administration, thereby providing an aqueous solution comprising fosaprepitant.

Fosaprepitant is converted in vivo to aprepitant. Thus, when administered to a patient, typically intravenously, fosaprepitant is converted to aprepitant.

### Treatment of NSCLC and/or Breast Cancer

Typically the patient to be treated is a mammal. Preferably the patient is a human.

Treatment may be curative or palliative in nature, i.e. it may aim at curing the patient, achieving complete or partial remission, alleviating or managing symptoms and/or side effects of the disease (without curing the patient) and/or increasing life expectancy.

The treatment may be an adjuvant therapy or a neo-adjuvant therapy, for example being combined with surgery, other chemotherapy strategies and/or radiotherapy. Alternatively, the treatment may be carried out in the absence of surgery, other chemotherapy strategies and/or radiotherapy.

The NSCLC to be treated may be metastatic or non-metastatic and may be resectable or unresectable. The NSCLC may also be refractory to conventional chemotherapies. The NSCLC may be selected from the sub-group of NSCLCs comprising squamous-cell carcinoma, large-cell carcinoma, adenocarcinoma, pleomorphic, carcinoid tumour, salivary gland carcinoma, unclassified carcinoma and c-SCLC.

It is also particularly preferred that the NSCLC comprises the molecular profile EGFR- / ALK- / ROS 1-. A EGFR- / ALK- / ROS1- molecular profile means that a cell in a NSCLC tumour to be treated has wild type EGFR, wild type ALK and wild type ROS1 genes (EGFR- / ALK- / ROS1-). In other words, the EGFR, ALK and ROS1 genes do not contain mutations, such as translocations, inversions, insertions, deletions, substitutions or point mutations, relative to a wild type reference sequence. A skilled person can readily assess whether or not EGFR, ALK and/or ROS1 is wild type. For example, the skilled person could compare the sequence of genomic DNA, mRNA and/or protein from a cell isolated from a NSCLC tumour respectively with genomic DNA, mRNA and/or protein from an isolated wild type reference cell, and thereby determine without difficulty whether a NSCLC cell has mutations compared to a wild type reference cell and therefore does not have a EGFR- / ALK- / ROS1- molecular profile. A NSCLC tumour may be classed as having a EGFR- / ALK- / ROS1- molecular profile if at least 1%, at least 2%, at least 5%, at least 10%, at least 20%, at least 30%, at least 40%, at least 50%, at least 60%, at least 70%, at least 80%, at least 90%, at least 95% or at least 99% of cells in the tumour have the EGFR- / ALK- / ROS 1- molecular profile.

In some embodiments, it is preferred that the NSCLC comprises the molecular profile EGFR- / ALK- / ROS 1- and: (i) would not be suitable for treatment using drugs directed against specific EGFR therapeutic targets, such as Erlotinib or Gefitinib; (ii) would not be suitable for treatment using drugs directed against the specific EGFR therapeutic targets, such as Crizotinib or Ceritinib; (iii) does not express high levels of PD1-PDL1 and/or has wild type or substantially wild type expression of PD1-PDL1; and/or (iv) would not be suitable for treatment using immunotherapy.

The breast cancer to be treated may be metastatic or non-metastatic and may be resectable or unresectable. The breast cancer may also be refractory to conventional chemotherapies. The breast cancer may be selected from the sub-group of breast cancers comprising stage 0, stage 1, stage 2, stage 3 or stage 4 breast cancer. In addition, or in the alternative, the breast cancer may be selected from the sub-group of breast cancers comprising DCIS, LCIS, invasive breast cancer, inflammatory breast cancer, paget disease of the breast, angiosarcoma or phyllodes tumour.

It is particularly preferred that the breast cancer comprises the triple negative molecular profile ER- / PR- / HER2-. A triple negative ER- / PR- / HER2- molecular profile means that a cell in a breast cancer tumour to be treated does not express, or substantially does not express all of an estrogen receptor, a progesterone receptor and HER2. A skilled person can readily assess whether or not an estrogen receptor, a progesterone receptor and/or HER2 are not expressed. For example, the skilled person could compare the expression level of an estrogen receptor, a progesterone receptor and HER2 with the respective expression levels of an estrogen receptor, a progesterone receptor and HER2 in a positive and/or negative control sample. Expression levels may be measured by assessing mRNA levels, for example using qRT PCR. In addition or alternatively, expression levels may be measured by assessing protein levels, for example using immunohistochemistry (IHC) and/or an ELISA. qRT PCR, IHC and/or ELISAs may be performed with test samples obtained from a suspected breast cancer biopsy. In addition or alternatively, qRT PCT and/or ELISAs may be performed with test samples obtained from serum, for example to assay cell free mRNA or cell free protein. In doing, so, the skilled person can thereby determine without difficulty whether or not a breast cancer has a triple negative ER- / PR- / HER2- molecular profile. A breast cancer may be classed as having a triple negative ER- / PR- / HER2- molecular profile if at least 1%, at least 2%, at least 5%, at least 10%, at least 20%, at least 30%, at least 40%, at least 50%, at least 60%, at least 70%, at least 80%, at least 90%, at least 95% or at least 99% of cells in the tumour have the ER- / PR- / HER2- molecular profile. Alternatively, a breast cancer may be classed as having a triple negative ER- / PR- / HER2- molecular profile if when measured by immunohistochemistry, for example according to the criteria defined by The College of American Pathologists in the document "Template for Reporting Results of Biomarker Testing of Specimens From Patients With Carcinoma of the Breast" (Version: Breast Biomarkers 1.4.0.0 Template Posting Date: February 2020, available at the url: https://documents.cap.org/protocols/cp-breast-biomarker-20-1400.pdf): (i) less than 1% of cells in the tumour demonstrate nuclear positive staining for ER (ER-); (ii) less than 1% of cells in the tumour demonstrate nuclear positive staining for PR (PR-); and (iii) in the tumour no HER2 staining is observed, or membrane staining of HER2 is incomplete, faint and/or barely perceptible within ≤ 10% of the tumour cells. The skilled person would readily be able to determine the nature of the immunohistochemistry staining.

In some embodiments, it is preferred that the breast cancer comprises the molecular profile ER- / PR- / HER2- and: (i) is not treated or suitable for treatment using a drug targeting an estrogen receptor such as tamoxifen; (ii) is not treated or suitable for treatment using a drug targeting a progesterone receptor; (iii) is not treated or suitable for treatment using a drug targeting HER such as trastuzumab; (iv) does not express high levels of PD1-PDL1 and/or has wild type or substantially wild type expression of PD1-PDL1; and/or (v) would not be suitable for treatment using immunotherapy.

In some embodiments, NSCLC as set out in this section, or Breast Cancer as set out in this section, is treated. In alternative embodiments, NSCLC as set out in this section and Breast Cancer as set out in this section is treated. When both NSCLC and Breast Cancer is treated, the efficacy of treatment of NSCLC and Breast Cancer when measured using a comparable metric (such as percentage reduction in the size of a tumour and/or percentage reduction in the number of cancerous cells) may be substantially the same or different for NSCLC and Breast Cancer. Based on their common general knowledge, the skilled person would readily be able to determine a comparable metric, and conduct any necessary tests to determine the efficacy of treatment of NSCLC and/or Breast Cancer.

### Pharmaceutical compositions

The present invention involves the use of a NK₁ inhibitor, which is preferably aprepitant or fosaprepitant, or a pharmaceutically acceptable salt thereof, for treating NSCLC having a EGFR- / ALK- / ROS1- profile. The present invention also involves the use of a NK₁ inhibitor, which is preferably aprepitant or fosaprepitant, or a pharmaceutically acceptable salt thereof, for treating breast cancer having a ER- / PR- / HER2- molecular profile. The present invention also involves the use of a NK₁ inhibitor, which is preferably aprepitant or fosaprepitant, or a pharmaceutically acceptable salt thereof, for treating: (i) NSCLC having a EGFR- / ALK- / ROS 1- profile; and (ii) breast cancer having a ER- / PR- / HER2- molecular profile. The NK₁ inhibitor is herein referred to as the "active ingredient".

In one aspect, the present invention provides a pharmaceutical composition which comprises a NK₁ inhibitor, for use in treating non-small cell lung carcinoma (NSCLC) wherein at least 1% of the cells in a NSCLC tumour to be treated have wild type EGFR, ALK and ROS 1 genes. In another aspect, the present invention provides a pharmaceutical composition which comprises a NK₁ inhibitor, for use in treating breast cancer wherein at least 1% of the cells in a breast cancer tumour to be treated have a ER- / PR- / HER2-molecular profile. In yet another aspect, the present invention provides a pharmaceutical composition which comprises a NK₁ inhibitor, for use in treating: (i) non-small cell lung carcinoma (NSCLC) wherein at least 1% of the cells in a NSCLC tumour to be treated have wild type EGFR, ALK and ROS1 genes; and (ii) breast cancer wherein at least 1% of the cells in a breast cancer tumour to be treated have a ER- / PR- / HER2- molecular profile. Pharmaceutical compositions according to the invention will typically further comprise one or more pharmaceutically acceptable excipients or carriers.

In general, administration of the pharmaceutical composition may be oral (as syrups, tablets, capsules, lozenges, controlled-release preparations, fast-dissolving preparations, etc.), by injection (subcutaneous, intradermal, intramuscular, intravenous, etc.), or by inhalation (as a dry powder, a solution, a dispersion, etc.).

The preferred route of administration will depend upon the specific active ingredient to be delivered, and a skilled person can easily choose an appropriate route. For example, aprepitant is preferably delivered orally, whereas fosaprepitant is preferably administered intravenously.

For oral administration, the pharmaceutical compositions of the present invention may take the form of, for example, tablets, lozenges or capsules prepared by conventional means with pharmaceutically acceptable excipients such as binding agents (e.g. pregelatinised maize starch, polyvinylpyrrolidone or hydroxypropyl methyl cellulose); fillers (e.g. lactose, microcrystalline cellulose or calcium hydrogenphosphate); lubricants (e.g. magnesium stearate, talc or silica); disintegrants (e.g. potato starch or sodium glycolate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles or preservatives. The preparations may also contain buffer salts, flavouring agents, colouring agents or sweetening agents, as appropriate.

For administration by injection, the pharmaceutical compositions typically take the form of an aqueous injectable solution. Examples of suitable aqueous carriers that may be employed in the injectable pharmaceutical compositions of the invention include water, buffered water and saline. In many cases, it will be preferable to include isotonic agents, for example, sugars, polyalcohols such as mannitol, sorbitol, or sodium chloride in the composition.

For administration by inhalation, the pharmaceutical composition may take the form of a dry powder, which will typically comprise the active ingredient and a carrier such as lactose, and be delivered via an inhaler. Alternatively, the pharmaceutical composition may for example be formulated as aqueous solutions or suspensions and be delivered as an aerosol from a pressurised metered dose inhaler, with the use of a suitable liquefied propellant. Suitable propellants include fluorocarbon or hydrogen-containing chlorofluorocarbon or mixtures thereof, particularly hydrofluoroalkanes.

Pharmaceutical compositions of the invention may be prepared by any suitable method known to those of skill in the art.

Pharmaceutical compositions of the invention may comprise additional active ingredients, such as an additional therapeutic or prophylactic agent intended, for example, for the treatment of the same condition or a different one, or for other purposes such as amelioration of side effects. In such situations, the active ingredients may be co-administered. When active ingredients are co-administered, they can be present either in a single pharmaceutical composition or in separate pharmaceutical compositions, including in separate pharmaceutical compositions optimized for administration either by the same mode or a different mode. For example, the active ingredients may both be administered intravenously, either in a single pharmaceutical composition or, more preferably, in separate pharmaceutical compositions.

However, it is generally preferred that the compositions of the invention do not contain any further active ingredients (i.e. the pharmaceutical compositions contain only a NK₁ inhibitor, which is preferably aprepitant or fosaprepitant, or a pharmaceutically acceptable salt thereof.

### Dosages and dosage regimes

Suitable dosages of the active ingredient used in the present invention may easily be determined by a skilled medical practitioner.

Actual dosage levels of the active ingredient in the pharmaceutical compositions of the present invention may be varied so as to obtain an amount of the active ingredient, which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

Appropriate doses may be determined by a physician. Appropriate doses are typically proportionate to the body weight of the subject. A suitable dosage may be determined by a skilled medical practitioner. Actual dosage levels may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the active ingredient employed, the route of administration, the time of administration, the rate of excretion of the active ingredient, the duration of the treatment, other drugs, compounds and/or materials used in combination with the particular compositions employed, the age, sex, weight, condition, general health and prior medical history of the patient being treated, and like factors well known to the skilled person.

A suitable dose of the active ingredient (i.e. NK₁ inhibitor such as aprepitant or fosaprepitant, or a pharmaceutically acceptable salt thereof), may be, for example, in the range of from about 5 mg/kg/day to about 120 mg/kg/day. The lower limit of the range may be 5, 7.5, 10, 12.5, 15, 20, 25, 30, 35, 40, 50, 75 or 100 mg/kg/day. The upper limit of the range may be 7.5, 10, 12.5, 20, 25, 30, 35, 40, 50, 75, 100 or 120 mg/kg/day. The upper and lower limits may be combined to arrive at a specific dosage range. For example, the specific dosage range of the active ingredient may be 5-100 mg/kg/day, 5-75 mg/kg/day, 5-50 mg/kg/day, 5-40 mg/kg/day, 5-35 mg/kg/day, 5-30 mg/kg/day, 5-25 mg/kg/day, 5-20 mg/kg/day, 5-15 mg/kg/day, 5-12.5 mg/kg/day, 5-10 mg/kg/day, 5-7.5 mg/kg/day, 7.5-100, 7.5-100 mg/kg/day, 7.5-75 mg/kg/day, 7.5-50 mg/kg/day, 7.5-40 mg/kg/day, 7.5-35 mg/kg/day, 7.5-30 mg/kg/day, 7.5-25 mg/kg/day, 7.5-20 mg/kg/day, 7.5-15 mg/kg/day, 7.5-12.5 mg/kg/day, 7.5-10 mg/kg/day, 10-100 mg/kg/day, 10-75 mg/kg/day, 10-50 mg/kg/day, 10-40 mg/kg/day, 10-35 mg/kg/day, 10-30 mg/kg/day, 10-25 mg/kg/day, 10-20 mg/kg/day, 10-15 mg/kg/day, 10-12.5 mg/kg/day, 12.5-100 mg/kg/day, 12.5-75 mg/kg/day, 12.5-50 mg/kg/day, 12.5-40 mg/kg/day, 12.5-35 mg/kg/day, 12.5-30 mg/kg/day, 12.5-25 mg/kg/day, 12.5-20 mg/kg/day, 12.5-15 mg/kg/day, 15-100 mg/kg/day, 15-75 mg/kg/day, 15-50 mg/kg/day, 15-40 mg/kg/day, 15-35 mg/kg/day, 15-30 mg/kg/day, 15-25 mg/kg/day, 15-20 mg/kg/day, 20-100 mg/kg/day, 20-75 mg/kg/day, 20-50 mg/kg/day, 20-40 mg/kg/day, 20-35 mg/kg/day, 20-30 mg/kg/day, 20-25 mg/kg/day, 25-100 mg/kg/day, 25-75 mg/kg/day, 25-50 mg/kg/day, 25-40 mg/kg/day, 25-35 mg/kg/day, 25-30 mg/kg/day, 30-100 mg/kg/day, 30-75 mg/kg/day, 30-50 mg/kg/day, 30-40 mg/kg/day, 30-35, 35-100 mg/kg/day, 35-75 mg/kg/day, 35-50 mg/kg/day, 35-40 mg/kg/day, 40-100 mg/kg/day, 40-75 mg/kg/day, 40-50 mg/kg/day, 50-100 mg/kg/day, or 50-75 mg/kg/day. In one preferred embodiment, the dose is between 5-35 mg/kg/day. In a more preferred embodiment, the dose is between 5-30 mg/kg/day. In an even more preferred embodiment, the dose is between 5-25 mg/kg/day. In an even more preferred embodiment, the dose is between 5-20 mg/kg/day. In an even more preferred embodiment, the dose is between 5-15 mg/kg/day. In an even more preferred embodiment, the dose is between 5-12.5 mg/kg/day. In an even more preferred embodiment, the dose is between 5-10 mg/kg/day. In one preferred embodiment, the does is between 10-35 mg/kg/day. In a more preferred embodiment, the dose is between 10-30 mg/kg/day. In an even more preferred embodiment, the does is between 10-25 mg/kg/day. In an even more preferred embodiment, the dose is between 10-20 mg/kg/day. In an even more preferred embodiment, the does is between 10-15 mg/kg/day. In one preferred embodiment, the does is between 5-35 mg/kg/day. In a more preferred embodiment, the dose is between 7.5-35 mg/kg/day. In an even more preferred embodiment, the does is between 10-35 mg/kg/day. In an even more preferred embodiment, the dose is between 12.5-35 mg/kg/day. In an even more preferred embodiment, the does is between 15-35 mg/kg/day. In an even more preferred embodiment, the does is between 20-35 mg/kg/day. In an even more preferred embodiment, the does is between 25-35 mg/kg/day. In an even more preferred embodiment, the does is between 30-35 mg/kg/day.

The unit mg/kg/day denotes that a specified amount of active ingredient is administered to a patient each day according to the number of kg body weight of the patient to be treated. For example, if 5 mg/kg/day of the active ingredient is to be administered to a patient weighing 80kg, then 400mg of the active ingredient would be administered per day. The active ingredient may be administered one, two, three, four or more times a day, in a total daily amount as indicated above.

It is a finding of the present invention that a profound anti-NSCLC effect may be observed when a NK₁ inhibitor is administered to a NSCLC patient subgroup where the NSCLC has a EGFR- / ALK- / ROS1- profile. It is also a finding of the present invention that a profound anti-triple negative breast cancer effect may be observed when a NK₁ inhibitor is administered to a breast cancer patient subgroup where the breast cancer has a triple negative ER- / PR- / HER2- molecular profile. Additionally, it is a finding of the present invention that: (i) a profound anti-NSCLC effect may be observed when a NK₁ inhibitor is administered to a NSCLC patient subgroup where the NSCLC has a EGFR- / ALK- / ROS 1- profile; and a profound anti-triple negative breast cancer effect may be observed when a NK₁ inhibitor is administered to a breast cancer patient subgroup where the breast cancer has a triple negative ER- / PR- / HER2- molecular profile.

Dosage regimens may be adjusted to provide the optimum desired response. For example, a single dose may be administered, several divided doses may be administered over time or the dose may be proportionally reduced or increased as indicated by the exigencies of the therapeutic situation. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Administration may be in single or multiple doses. Multiple doses may be administered via the same or different routes and to the same or different locations. Dosage and frequency may vary depending on the half-life of the drugs in the patient and the duration of treatment desired.

NK₁ inhibitors, such as aprepitant and fosaprepitant, and pharmaceutically acceptable salts thereof, are currently approved for use in treating nausea and vomiting in patients, including those suffering from cancer who may also be suffering from nausea and vomiting as a result of chemotherapy and/or surgery. It is thus preferred in the present invention that the NK₁ inhibitor is not prescribed and/or administered to the patient for the purpose of treating nausea and vomiting, but instead is prescribed and/or administered to the patient for treating NSCLC and/or triple negative breast cancer.

The present invention is explained in more detail in the following by referring to the Examples, which are not to be construed as limitative.

### EXAMPLES

### Example 1 - Xenograft Mouse Safety Model

Fosaprepitant dimeglumine salt (CAS No 265121-04-8) was purchased from Selleck Chemicals and administered intraperitoneally at 30, 60 and 100 mg/kg dose, once a day. Fosaprepitant is a pro-drug available for parenteral administration that in the body, once administered, becomes aprepitant.

3LL cells (0.6 x 10⁶ per mouse) or B16F10 cells (1 x 10⁶ per mouse) were subcutaneously injected into the right flank of C57BL/6J mice (Charles River) with matrigel (Matrigel Membrane Matrix, Corning) at 50% v/v. Tumour growth was evaluated every 2/3 days. Animal vital parameters were monitored daily. When the tumours reached an average volume of 100-150 mm³, mice were randomized into cohorts of 6 animals each and treatments were administered for 7-9 days. Groups and regimens for each experiment are described in Table 1. Animals of the control group were dosed with equal volume of vehicle. Animals were euthanized according to institutional guidelines and tumour samples were excised. Tumours were snap-frozen in OCT medium (Sakura Tissue Tek) or fixed in PFA and embedded in paraffin. Several internal organs were also excised and fixed in PFA for future studies.

**Table 1 Dose of aprepitant administered (mg/kg/day) and the mean of body weight in each case and its corresponding standard deviation.**

| **Dose Fosaprepitant** | | **Body weight day 1** | | **Body weight day 3** | | **Body weight day 8** | |
|---|---|---|---|---|---|---|---|
| | N | Mean | SD | Mean | SD | Mean | SD |
| **Control** | 20 | 19.76 | 0.96 | 19.79 | 1.12 | 21.62 | 1.31 |
| **30 mg / kg of body weight / day** | 16 | 19.5 | 0.97 | 19.16 | 0.99 | 20.89 | 1.53 |
| **60 mg / kg of body weight / day** | 3 | 19.56 | 0.30 | 19.07 | 0.85 | 20.62 | 0.70 |
| **100 mg / kg of body weight / day** | 4 | 19.67 | 0.51 | 18.95 | 0.39 | 20.62 | 0.55 |

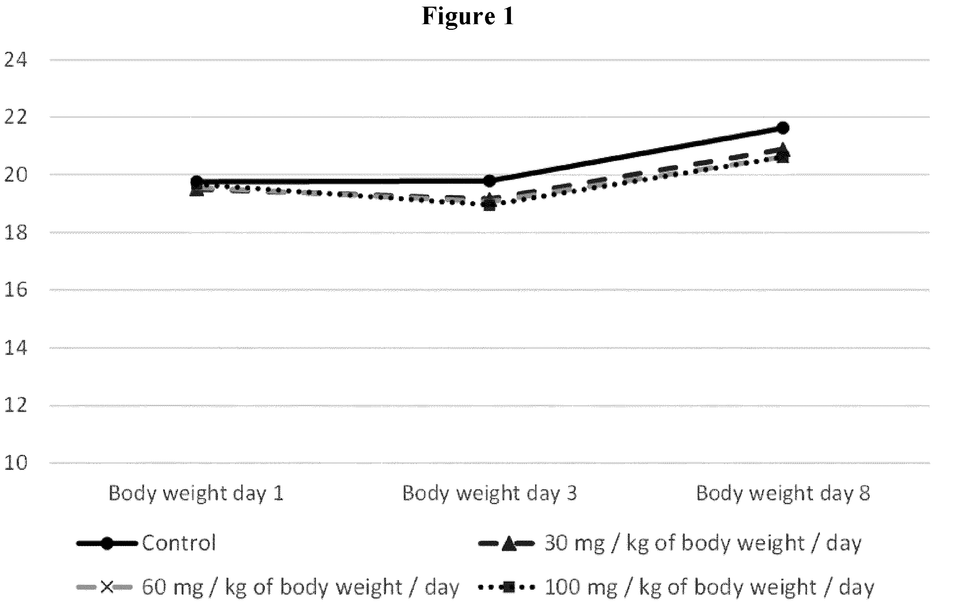

Figure 1 shows a graphical representation of the body weight mean, on the "Y" axis, in relation to the day, on the "X" axis, for each dose of aprepitant administered (mg/kg/day). There was no significant reduction in body weight following administration of aprepitant. Further, all animals were subjected to autopsy and the organs explored microscopically revealing no significant signs of toxicity.

Thus, it has been shown that the use of a NK₁ inhibitor such as aprepitant, even at a dose of 100 mg / kg of body weight per day, is safe since no relevant alterations were observed in the anthropometric parameters (such as body weight) or in the histological study of the main organs.

### Example 2 - Cell Culture Experimental Procedures

EGFR, ALK and ROS1 are key molecular factors that may be used to determine pathological classification, prognosis, therapeutics and behaviour in patients with lung cancer. EGFR, ALK and ROS 1 are biomarkers routinely analysed in the clinical practice of the management of patients with NSCLC. NSCLC with molecular profile EGFR- / ALK- / ROS1- presents a better response to treatment with the NK₁ inhibitor aprepitant, compared to lung cancer with different profiles (in particular, EGFR+ / ALK- / ROS1- and EGFR- / ALK+ / ROS 1-, in which EGFR and ALK respectively contain mutations relative to their wild type gene). Commercial lines of lung cancer were used as shown in Table 2 together with their molecular profile. The molecular profile of the cancer cell lines was determined by sequencing and can be consulted in the Cancer Cell Line Encyclopaedia database of public access available at the url: https://portals.broadinstitute.org/ccle

**Table 2 Commercial lines of lung cancer used, together with the molecular profile of each cell line.**

| **Tumour Cell Line** | **Molecular Profile** |
|---|---|
| A549 | EGFR-/ALK-/ROS1- |
| H358 | EGFR-/ALK-/ROS1- |
| H441 | EGFR-/ALK-/ROS1- |
| H460 | EGFR-/ALK-/ROS1- |
| H520 | EGFR-/ALK-/ROS1- |
| HCC4006 | EGFR+/ALK-/ROS1- |
| H322 | EGFR-/ALK+/ROS1- |
| H332 | EGFR-/ALK+/ROS1- |

All cells were cultured in wells under vendor-recommended conditions and exposed to different concentrations of aprepitant for 48 hours. In each case a group of wells of cells were used to which no treatment was applied and which were considered as controls. At 48 hours the number of cells was measured.

Eight cell lines of human were used for the study of NK₁ inhibitors (Table 3). All of them were maintained in culture in their respective culture media (Gibco,Thermo Fisher Scientific) supplemented with 10% foetal bovine serum (FBS)(Gibco, Thermo Fisher Scientific), 2 mM L-Glutamine (Gln)(Sigma-Aldrich, Merk), and Peni-Strep (P-S)(50 U/ml and 50 µl/ml; Gibco, Thermo Fisher Scientific) in a humidified incubator at 37°C and 5% CO₂. Cell culture media used are Dulbecco's Modified Eagle Medium (DMEM), and RPMI Medium 1640 (RPMI-1640).

**Table 3 Further information on commercial cell lines and the culture media used.**

| **Cell line** | **Disease** | **Cell culture media** | **Supplements** |
|---|---|---|---|
| A549 | Lung carcinoma | DMEM | 10% FBS, Gln, P-S |
| H358 | Non-small cell lung cancer/carcinoma | RPMI-1640 | 10% FBS, Gln, P-S |
| H441 | Lung papillary adenocarcinoma | RPMI-1640 | 10% FBS, Gln, P-S |
| H460 | Large cell lung carcinoma | RPMI-1640 | 10% FBS, Gln, P-S |
| H520 | Lung squamous cell adenocarcinoma | RPMI-1640 | 10% FBS, Gln, P-S |
| HCC4006 | Lung adenocarcinoma | RPMI-1640 | 10% FBS, Gln, P-S |
| H322 | Lung papillary adenocarcinoma | RPMI-1640 | 10% FBS, Gln, P-S |
| H332 | Lung adenocarcinoma | RPMI-1640 | 10% FBS, Gln, P-S |

To measure cell viability, the cytotoxicity of drugs in culture was evaluated using the 3-(4,5-Dimethyl-2-thiazolyl)-2,5-diphenyl-2H-tetrazolium bromide (MTT) reduction assay. This assay assesses cell viability through the indirect measurement of the activity of reductases that convert yellow soluble MTT into purple insoluble formazan salts. As MTT can only be reduced when these enzymes are active, the reaction is used as an indicator of the cell metabolic competence and, therefore, of viability.

A variable number of cells (depending on the cell line) were seeded in 96 multi-well plates and grown during 24 hours. The following day, the culture medium was aspirated and fresh culture medium with the drug dissolved was added to the wells. After 24 hours or 48 hours cell viability was measurement by adding 10 µl of a 5 mg/ml MTT solution (M5655; Sigma-Aldrich) and 100 µl of HCl 0.01M-SDS 10% solution 4 hours later. After 24 hours, the absorbance was measured at 595 nm, using a multi-well plate reader (iMark, Bio-rad). Each condition was tested 5 times, obtaining 5 values of absorbance. All sample measurements were corrected using the blank (culture medium without cells) mean absorbance value, and normalized to positive control to determinate relative cell viability for each condition at 24 and 48 hours.

### Example 3 - Efficacy of NK₁ inhibitors in treating NSCLC

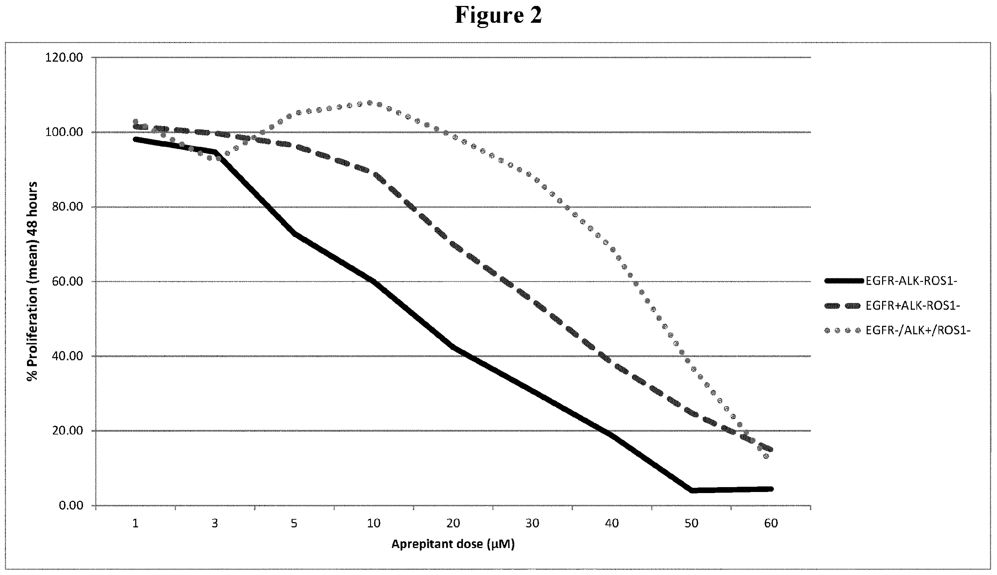

Table 4 shows the dose of aprepitant administered (µM) and the molecular profile together with the mean proliferation in each case and its corresponding standard deviation. This data is shown graphically in Figure 2.

**Table 4 Dose of aprepitant administered (µM), the molecular profile and mean proliferation**

| | **EGFR-ALK-ROS1-(EGFR-/ALK-/ROS1-)** | | **EGFR+ALK-ROS1-(EGFR+/ALK-/ROS1-)** | | **EGFR-/ALK+/ROS1-** | |
|---|---|---|---|---|---|---|
| **Aprepitant Dose (µM)** | Mean | Std. Deviation | Mean | Std. Deviation | Mean | Std. Deviation |
| **1** | 98.09 | 4.16 | 101.53 | 5.47 | 102.88 | 5.75 |
| **3** | 94.71 | 5.18 | 99.73 | 6.62 | 92.41 | 6.26 |
| **5** | 72.82 | 10.43 | 96.4 | 2.44 | 105.07 | 5.26 |
| **10** | 59.95 | 5.82 | 89.14 | 1.93 | 108.06 | 9.27 |
| **20** | 42.37 | 9.26 | 69.95 | 1.16 | 99 | 7.81 |
| **30** | 30.66 | 7.06 | 54.9 | 1.96 | 88.22 | 15.24 |
| **40** | 18.72 | 6.51 | 38.12 | 0.87 | 68.94 | 11.06 |
| **50** | 4.05 | 3.71 | 24.82 | 6.79 | 37.3 | 23.35 |
| **60** | 4.40 | 5.83 | 14.95 | 1.27 | 11.67 | 18.48 |

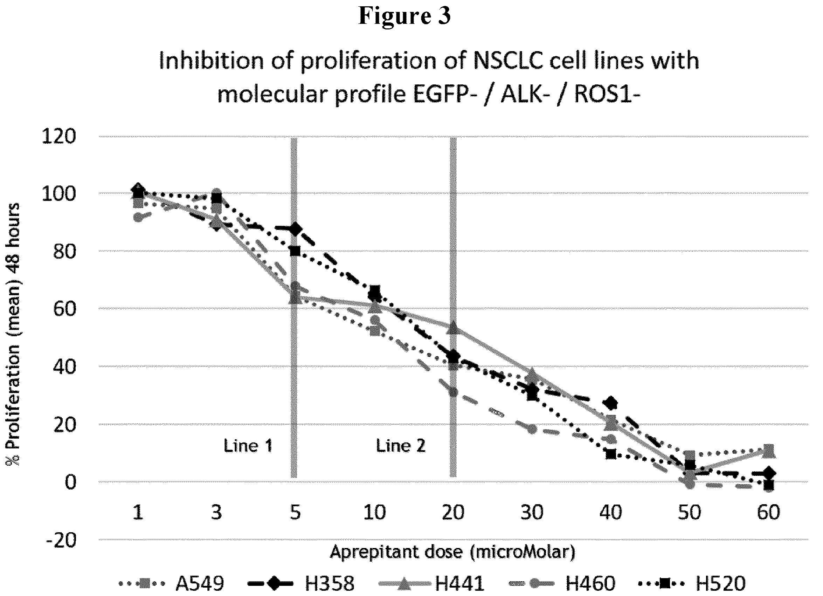

Tables 5 and 6 show the dose of aprepitant administered (µM) in the indicated cell lines, all with molecular profile EGFR- / ALK- / ROS1-, together with the mean proliferation in each case and its corresponding standard deviation. This data is shown graphically in Figure 3.

**Table 5 Dose of aprepitant administered (µM) in the indicated cell line and mean proliferation**

| **Cancer Cell Line** | **A549** | | **H358** | | **H441** | |
|---|---|---|---|---|---|---|
| **Aprepitant Dose (µM)** | Mean | Std. Deviation | Mean | Std. Deviation | Mean | Std. Deviation |
| **1** | 96.56 | 3.47 | 101.3 | 1.65 | 100.6 | 1.62 |
| **3** | 94.80 | 3.51 | 89.28 | 1.17 | 90.97 | 3.22 |
| **5** | 64.40 | 6.77 | 87.69 | 4.19 | 64.10 | 4.03 |
| **10** | 52.17 | 2.16 | 64.12 | 1.72 | 61.08 | 2.96 |
| **20** | 40.48 | 2.22 | 43.44 | 1.67 | 53.64 | 13.45 |
| **30** | 35.59 | 1.53 | 32.09 | 0.98 | 37.56 | 3.05 |
| **40** | 21.55 | 3.68 | 27.23 | 0.36 | 20.50 | 2.75 |
| **50** | 9.17 | 2.99 | 3.10 | 0.44 | 3.14 | 1.22 |
| **60** | 11.36 | 0.70 | 2.86 | 0.44 | 10.83 | 0.75 |

**Table 6 Dose of aprepitant administered (µM) in the indicated cell line and mean proliferation**

| **Cancer Cell Line** | **H460** | | **H520** | |
|---|---|---|---|---|
| **Aprepitant Dose (µM)** | Mean | Std. Deviation | Mean | Std. Deviation |
| **1** | 91.72 | 0.15 | 100.27 | 2.50 |
| **3** | 100.26 | 2.57 | 98.27 | 4.65 |
| **5** | 67.81 | 1.54 | 80.08 | 3.68 |
| **10** | 56.01 | 2.37 | 66.40 | 3.40 |
| **20** | 31.24 | 1.08 | 43.07 | 2.33 |
| **30** | 18.27 | 1.01 | 29.80 | 0.69 |
| **40** | 14.74 | 1.49 | 9.59 | 1.54 |
| **50** | -0.95 | 0.28 | 5.78 | 1.41 |
| **60** | -1.95 | 0.15 | -1.07 | 0.42 |

The results obtained in vitro by culturing commercial cell lines of non-small cell type lung cancer/carcinoma, both with an EGFR- / ALK- / ROS1- profile, and with an EGFR+ / ALK- / ROS 1- profile, as with the EGFR- / ALK+ / ROS 1- profile, discussed above and shown in Table 4 and Figure 2, show that the specific subgroup of NSCLC with the EGFR- / ALK- / ROS 1- profile has a clearly improved response to treatment with the NK₁ inhibitor aprepitant. The subgroup of patients with NSCLC with an EGFR- / ALK- / ROS 1- molecular profile constitutes a subgroup of patients with an especially poor prognosis compared to patients whose tumours present a molecular profile EGFR+ / ALK-/ ROS1- or EGFR- / ALK+ / ROS1-. This is because patients whose lung cancers present an EGFR+ / ALK- / ROS 1- molecular profile are candidates for treatment with drugs directed against specific EGFR therapeutic targets, such as Erlotinib or Gefitinib that can provide an improvement in overall survival and in progression-free survival. Likewise, patients whose lung cancers present an EGFR- / ALK+ / ROS1- or ROS1+ molecular profile are candidates for treatment with drugs directed against the specific EGFR therapeutic targets, such as Crizotinib or Ceritinib, which likewise provide an improvement in survival in these patients. Patients whose lung cancers present an EGFR- / ALK- / ROS 1- profile are not candidates for treatment with these targeted treatments and would only be candidates for immunotherapy treatment if they present high PD1-PDL1 expression. It is, however, in these patients that a striking improvement in the response to treatment with aprepitant was observed and therefore these patents represent a promising subset of patient for treating with a NK₁ inhibitor, providing an effective therapeutic tool for NSCLC patient who are not candidates for or have become resistant to immunotherapy treatment and therefore representing a subset of NSCLC patients with an especially poor prognosis. Further, the results presented in Tables 5 and 6 and Figure 3 show that the response observed in NSCLC cases with an EGFR- / ALK- / ROS1- profile is quite homogeneous, regardless of the cell line used, further supporting the use of a NK₁ inhibitor to treat successfully NSCLC with the molecular profile EGFR- / ALK- / ROS1-. Specifically, the lung cancer cell lines A549, H358, H441, H460 and H520, all with EGFR- / ALK- / ROS1- profile, have been tested.

It has been shown that daily dosing of aprepitant from 625 to 875 mg would support plasma trough level of 2.65 µg/mL (equivalent to 5 µM) and that a clinical dose of 1140 mg/day can be extrapolated to a concentration of µM total (free + bound). In Figure 3, it can be seen that at a concentration of 5 µM (corresponding to a daily dose of 625 to 875 mg / day of aprepitant) in most cell lines an inhibition of proliferation of around the 20% (80% proliferation compared to control). Figure 3, also shows that at a concentration of 20 µM (corresponding to a daily dose of 1140 mg/day of aprepitant) indicates a growth inhibition of 60% (80% proliferation with respect to the control). Therefore, the dose to be used in the case of the subgroup of patients with lung cancer and in particular the subtype of NSCLC with a molecular profile EGFR- / ALK- / ROS1- should be established between 625 and 1000 mg / day since in this range of doses a significant increase of the effect is observed with respect to the dose used, although in principle higher doses could be used. The effect approaching 60% was seen as the dose approaches 1000 mg/day (12.5 mg/day for an adult of 80 kg body weight).

Thus, it has been shown that:
1. NK₁ inhibitors show a better effect when administered to patients with NSCLC having the molecular profile EGFR- / ALK- / ROS-;
2. this effect is appreciable at a concentration in particular ranging between 5 and 20 mg per day which corresponds to a dose ranging from approximately 625 to 1000 mg / day (7.8 to 12.5 mg/day for an adult of 80 kg body weight);
3. NK₁ inhibitors are safe at doses up to at least 100 mg/kg/day and no macroscopic or microscopic signs of toxicity are seen at these doses, in animal models and in the safety data recorded in regulatory agencies; and
4. NK₁ inhibitors are a good treatment opportunity for NSCLC, in particular in NSCLC patients having the molecular profile EGFR- / ALK- / ROS-, who currently have few or no treatment opportunities and a very poor prognosis.

### Example 4 - Administration of a NK₁ inhibitor inhibits growth of primary breast cancer cells in a Xenograft Mouse Model

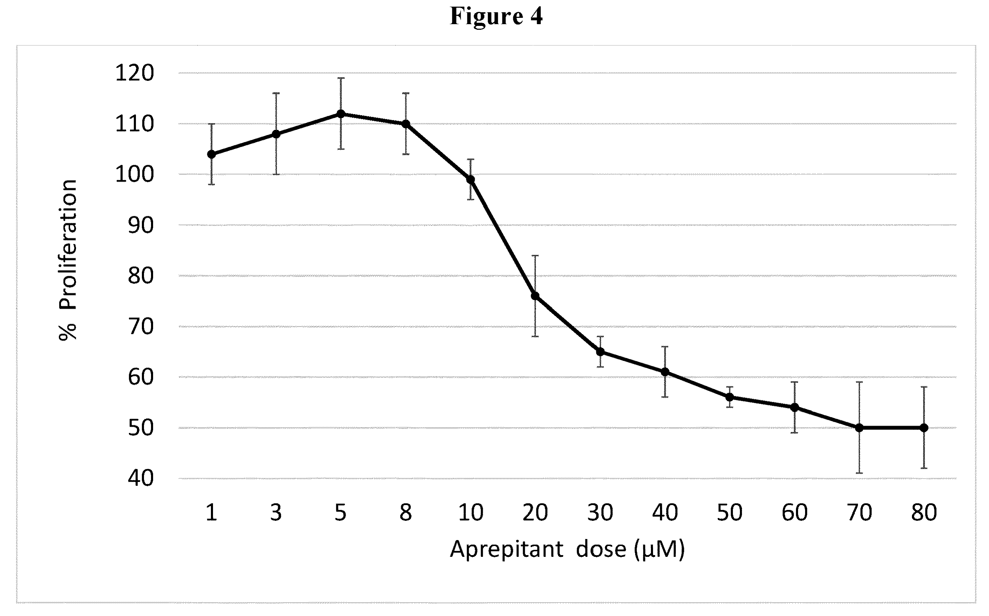

Immunocompromised female mice, 5-6 weeks of age were used, nu/nu Balb/c nude mice, provided by Harlan Iberica Barcelona (Spain). They were kept at 24° C and in sterile conditions with food and water "ad libitum". They were injected with 2×10⁷ tumour cells, corresponding to human primary triple negative breast carcinoma (i.e. tumour cells having a ER- / PR- / HER2- molecular profile) in 200 µl of PBS subcutaneous route. The tumour size was measured and the mice were evaluated for their state of health and weight daily. The mice were randomised in groups when the tumours reached a volume of 75 mm³. One of the groups was treated with 200 µl of placebo (control group) and the eight remaining groups with doses of 1 to 100 mg per kilogram of weight and day of the NK₁ inhibitor aprepitant. Five animals were treated per group, during 28 days. All the mice were sacrificed at the end of the experiment. The results are presented in Table 7 and in Figure 4.

**Table 7 Triple negative human primary breast carcinoma proliferation in the xenograft model.**

| **Aprepitant Dose (mg/kg/day)** | **Mean Proliferation** | **SD** |
|---|---|---|
| **1** | 104 | 6 |
| **3** | 108 | 8 |
| **5** | 112 | 7 |
| **8** | 110 | 6 |
| **10** | 99 | 4 |
| **20** | 76 | 8 |
| **30** | 65 | 3 |
| **40** | 61 | 5 |
| **50** | 56 | 2 |
| **60** | 54 | 5 |
| **70** | 50 | 9 |
| **80** | 50 | 8 |

It is clear from this data that the administration of a NK₁ inhibitor such as aprepitant reduces triple negative human primary breast carcinoma proliferation in a xenograft model in a dose-dependent fashion.

### Example 5 - Administration of a NK₁ inhibitor preferentially inhibits growth of triple negative breast cancer cells

Breast cancer with molecular profile ER- / PR- / HER2- presents a better response to treatment with a NK₁ inhibitor such as aprepitant, than breast cancer with different profiles (e.g. ER- / PR- / HER2+; ER+ / PR+ / HER2-; and ER+ / PR+ / Her2+).

For this example, the cell lines shown in Table 8 were tested. Table 8 also shows the molecular profile of each one of the cell lines.

**Table 8 Breast cancer cell lines tested and their molecular profile**

| **Tumour Cell Line** | **Molecular Profile** |
|---|---|
| BT474 | ER+ / PR+ / HER2+ |
| MCF7 | ER+ / PR+ / HER2- |
| MDAMB231 | ER- / PR- / HER2- |
| MDAMB435 | ER- / PR- / HER2- |
| MDAMB468 | ER- / PR- / HER2- |
| T47D | ER+ / PR+ / HER2- |
| SkBr3 | ER- / PR- / HER2+ |

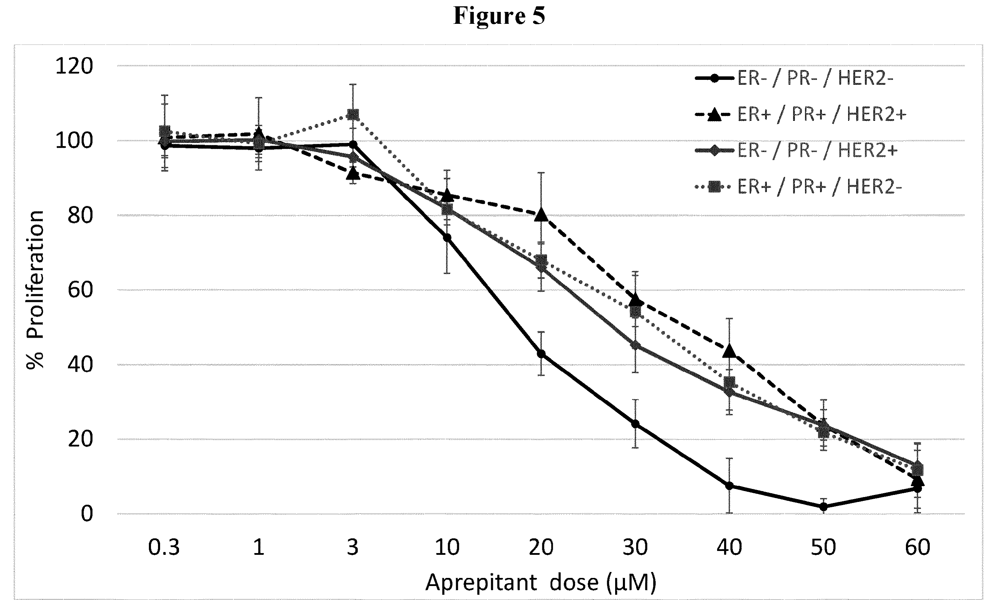

All cells were cultured in wells under vendor-recommended conditions and exposed to different concentrations of aprepitant for 48 hours. In each case a group of wells of cells were used to which no treatment was applied and which were considered as controls. At 48 hours the number of cells was measured. Results are presented in Table 9 and Figure 5.

To measure cell viability, the cytotoxicity of drugs in culture was evaluated using the 3-(4,5-Dimethyl-2-thiazolyl)-2,5-diphenyl-2H-tetrazolium bromide (MTT) reduction assay. This assay assesses cell viability through the indirect measurement of the activity of reductases that convert yellow soluble MTT into purple insoluble formazan salts. As MTT can only be reduced when these enzymes are active, the reaction is used as an indicator of the cell metabolic competence and, therefore, of viability.

A variable number of cells (depending on the cell line) were seeded in 96 multi-well plates and grown during 24 hours. The following day, the culture medium was aspirated and fresh culture medium with the drug dissolved was added to the wells. After 24 hours or 48 hours cell viability was measurement by adding 10 µl of a 5 mg/ml MTT solution (M5655; Sigma-Aldrich) and 100 µl of HCl 0.01M-SDS 10% solution 4 hours later. After 24 hours, the absorbance was measured at 595 nm, using a multi-well plate reader (iMark, Bio-rad). Each condition was tested 5 times, obtaining 5 values of absorbance. All sample measurements were corrected using the blank (culture medium without cells) mean absorbance value, and normalized to positive control to determinate relative cell viability for each condition at 24 and 48 hours.

**Table 9 Dose of aprepitant administered, the molecular profile, mean proliferation, and standard deviation**

| | **Molecular Profile** | | | | | | | |
|---|---|---|---|---|---|---|---|---|
| | **ER- / PR- / HER2-** | | **ER+ / PR+ / HER2+** | | **ER- / PR- / HER2+** | | **ER+ / PR+ / HER2-** | |
| **Aprepitant (µM)** | Mean | SD | Mean | SD | Mean | SD | Mean | SD |
| **0.3** | 98.63 | 3.23 | 100.88 | 8.93 | 99.8 | 3.81 | 102.47 | 9.67 |
| **1** | 97.94 | 3.52 | 101.83 | 9.68 | 100.23 | 3.84 | 99.16 | 3.74 |
| **3** | 98.98 | 4.36 | 91.36 | 2.89 | 95.65 | 2.68 | 106.96 | 8.14 |
| **10** | 74.02 | 9.55 | 85.45 | 6.65 | 81.8 | 8.02 | 81.55 | 4.13 |
| **20** | 42.89 | 5.83 | 80.23 | 11.1 | 65.97 | 6.36 | 67.98 | 4.79 |
| **30** | 24.11 | 6.43 | 57.51 | 7.39 | 45.14 | 7.31 | 54.13 | 9.79 |
| **40** | 7.53 | 7.28 | 43.65 | 8.62 | 32.62 | 6 | 35.22 | 7.46 |
| **50** | 1.89 | 2.17 | 23.8 | 4.09 | 23.78 | 6.75 | 21.79 | 3.66 |
| **60** | 6.77 | 6.44 | 9.29 | 7.77 | 12.87 | 5.79 | 11.7 | 7.26 |

It is clear from this data that the administration of a NK₁ inhibitor such as aprepitant reduces triple negative human primary breast carcinoma proliferation in culture relative to at least breast carcinomas having the molecular profile ER+ / PR+ / HER2+; ER- / PR- / HER2+; and ER+ / PR+ / HER2-, and that this reduction is dose dependent.

It has been shown that daily dosing of aprepitant from 625 to 875 mg would support plasma trough level of 2.65 µg/mL (equivalent to 5 µM) and that a clinical dose of 1140 mg/day can be extrapolated to a concentration of µM total (free + bound). In Figure 5, a concentration of 20 µM of aprepitant (corresponding to a daily dose of 1140 mg/day of aprepitant) indicates a growth inhibition of ~60%. Therefore, the dose to be used in the case of the subgroup of patients with triple negative breast cancer may be established around 1000 mg / day since in this range of doses a significant increase of the effect is observed with respect to the dose used compared to breast cancers not having a triple negative molecular profile. However, in principle higher doses could be used as the preferential inhibition of triple negative breast cancer versus other type of breast cancers tested was also seen at all doses tested above 20 µM of aprepitant.

Thus, it has been shown that:
1. NK₁ inhibitors show a better effect when administered to patients with breast cancer having the triple negative molecular profile ER- / PR- / HER2-;
2. this effect is appreciable at a concentration in particular ranging between 5 and 20 mg per day which corresponds to a dose ranging from approximately 625 to 1000 mg / day (7.8 to 12.5 mg/day for an adult of 80 kg body weight), but also at higher concentrations tested;
3. NK₁ inhibitors are safe at doses up to at least 100 mg/kg/day and no macroscopic or microscopic signs of toxicity are seen at these doses, in animal models and in the safety data recorded in regulatory agencies; and
4. NK₁ inhibitors are a good treatment opportunity for triple negative breast cancer, which at present has fewer treatment opportunities and a very poor prognosis.

### Example 6 - Determining the status of ER, PR and HER2

To determine the status of the Estrogen Receptors (ER), Progesterone Receptors (PR) and HER2, a cell block was made with 1 million cells from each of the cell lines. For this, cells were fixed in 4% buffered formalin, dehydrated and embedded in paraffin, creating a cell block. Such paraffin blocks were cut on a microtome to a thickness of 5 microns, which were placed on slides suitable for performing immunohistochemistry. Subsequently, samples were dewaxed by immersion in xylene and then were hydrated through a series of solutions containing decreasing concentrations of ethanol, to be finally immersed in water. More specifically, the immunohistochemical study was performed with the following reagents (Master Diagnóstica S.L.): Dewaxing and antigen recovery buffers, prediluted ready-to-use antibodies and Master Polimer Plus Detection System kit (MAD-000237QK). Automated dewaxing, hydration and unmasking were performed in pT module (95 ° C, 20 minutes). Immunostaining was performed automatically in the Autostainer 480 immunostainer (Thermo Scientific). Finally, hematoxylin counterstaining, dehydration, rinsing and mounting on a histological slide was performed. To ensure the functioning of the diagnostic antibodies, an external control with known expression, previously validated, was included in each preparation.

For the study of HER2 (C-erbB-2 / HER2 / NEU) the anti-human c-erbB2 Antibody (Master Diagnóstica; Clone SP3; catalog number MAD-000308QD) was used. For the study of estrogen receptors (ER), the anti-human estrogen receptor antibody (Master Diagnóstica; Clone SP1; catalog number MAD-000306QD) was used. For the study of progesterone receptors (PR), the anti-human progesterone receptor antibody (Master Diagnóstica; Clone 16; catalog number MAD-000670QD) was used.

The process was carried out under the accreditation 112 / ME2161 of ENAC (National Accreditation Entity of Spain; Website www.enac.es). The National Accreditation Entity (ENAC) is, according to Royal Decree 1715 of 20101, the only Spanish institution endowed with public power to grant accreditations in accordance with the provisions of European Regulation (EC) No. 765/20082.

Immunohistochemical results were evaluated according to the criteria defined by The College of American Pathologists in the document "Template for Reporting Results of Biomarker Testing of Specimens From Patients With Carcinoma of the Breast" (Version: Breast Biomarkers 1.4.0.0 Template Posting Date: February 2020, available at the url: https://documents.cap.org/protocols/cp-breast-biomarker-20-1400.pdf) which includes content from the updated CAP / ASCO ER / PgR Guideline (2020). For Estrogen Receptor (ER) status, the following values were considered: Positive (more than 10% of cells demonstrate nuclear positivity); Low Positive (1-10% of cells demonstrate nuclear positivity); and Negative (less than 1% demonstrate nuclear positivity). For Progesterone Receptor (PR) status, the following values were considered: Positive (more than 1% of cells demonstrate nuclear positivity); and Negative (less than 1% of cells demonstrate nuclear positivity). For HER2 status, the following values were considered: Negative (Score 0; No staining observed or Membrane stating that is incomplete and is faint / barely perceptible and within ≤ 10% of tumour cells), Negative (Score 1+; Incomplete membrane staining that is faint / barely perceptible and within > 10% of tumour cells), Equivocal (Score 2+; Weak to moderate complete membrane staining in > 10% of tumour cells or Complete membrane staining that is intense but within ≤10% of tumour cells) and Positive (Score 3+; Complete membrane staining that is intense and > 10% of tumour cells)."

The following are additional aspects of the present invention:
1. A pharmaceutical composition which comprises a NK₁ inhibitor, for use in treating:
   (a) non-small cell lung carcinoma (NSCLC) wherein at least 1% of the cells in a NSCLC tumour to be treated have wild type EGFR, ALK and ROS1 genes; and/or
   (b) breast cancer wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry.
2. The pharmaceutical composition for use according to 1, wherein the NK₁ inhibitor is aprepitant, fosaprepitant, netupitant, maropitant, vestipitant, casopitant, vofopitant, ezlopitant or lanepitant, or a pharmaceutically acceptable salt thereof.
3. The pharmaceutical composition for use according to 2, wherein the NK₁ inhibitor is aprepitant or fosaprepitant, or pharmaceutically acceptable salt thereof
4. The pharmaceutical composition for use according to 3, wherein the NK₁ inhibitor is aprepitant.
5. The pharmaceutical composition for use according to 3, wherein the NK₁ inhibitor is fosaprepitant dimeglumine.
6. A NK₁ inhibitor as defined in any one of 1 to 5, for use in treating:
   (a) NSCLC, wherein at least 1% of the cells in a NSCLC tumour to be treated have wild type EGFR, ALK and ROS1 genes; and/or
   (b) breast cancer, wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry.
7. A method of treating a patient suffering from:
   (a) NSCLC wherein at least 1% of the cells in a NSCLC tumour to be treated have wild type EGFR, ALK and ROS1 genes; and/or
   (b) breast cancer wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry;
   which method comprises administering to said patient a NK₁ inhibitor as defined in any one of 1 to 5.
8. Use of a NK₁ inhibitor as defined in any one of 1 to 5 in the manufacture of a medicament for the treatment of:
   (a) NSCLC, wherein at least 1% of the cells in a NSCLC tumour to be treated have wild type EGFR, ALK and ROS1 genes; and/or
   (b) breast cancer, wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry.
9. The pharmaceutical composition for use according to any one of 1 to 5, NK₁ inhibitor for use according to 6, the method according to 7, or use according to 8, wherein: (i) for a tumour that does not express estrogen receptor, less than 1% of cells in the tumour demonstrate nuclear positive staining for the estrogen receptor;
   (ii) for a tumour that does not express progesterone receptor less than 1% of cells in the tumour demonstrate nuclear positive staining for the progesterone receptor; and
   (iii) for a tumour that does not express HER2, HER2 staining is not observed in the tumour, or membrane staining of HER2 is incomplete, faint and/or barely perceptible within ≤ 10% of the tumour cells.
10. The pharmaceutical composition for use according to any one of 1 to 5 or 9, NK₁ inhibitor for use according to 6 or 9, the method according to 7 or 9, or use according to 8 or 9, wherein the NK₁ inhibitor is administered or used at a dose between 5 and 100 mg/kg/day, preferably 5 and 50 mg/kg/day.
11. The pharmaceutical composition for use, NK₁ inhibitor for use, method or use according to 9 or 10, wherein the NK₁ inhibitor is administered or used at a dose between 5 and 35 mg/kg/day, preferably between 5 and 25 mg/kg/day, more preferably between 5 and 15 mg/kg/day, most preferably between 5 and 10 mg/kg/day.
12. The pharmaceutical composition for use, NK₁ inhibitor for use, method or use according to 9 or 10, wherein the NK₁ inhibitor is administered or used at a dose between 5 and 35 mg/kg/day, preferably between 10 and 35 mg/kg/day, more preferably between 15 and 35 mg/kg/day, most preferably between 25 and 35 mg/kg/day.

## Claims

1. A pharmaceutical composition which comprises a NK₁ inhibitor, for use in treating:
breast cancer, wherein at least 1% of the cells in a breast cancer tumour to be treated do not express estrogen receptor and do not express progesterone receptor,
and at least 10% of the cells in a breast cancer tumour to be treated do not express HER2, wherein expression of estrogen receptor, progesterone receptor and HER2 is measured by immunohistochemistry.

2. The pharmaceutical composition for use according to claim 1, wherein the NK₁ inhibitor is aprepitant, fosaprepitant, netupitant, maropitant, vestipitant, casopitant, vofopitant, ezlopitant or lanepitant, or a pharmaceutically acceptable salt thereof.

3. The pharmaceutical composition for use according to claim 2, wherein the NK₁ inhibitor is aprepitant or fosaprepitant, or pharmaceutically acceptable salt thereof

4. The pharmaceutical composition for use according to claim 3, wherein the NK₁ inhibitor is aprepitant.

5. The pharmaceutical composition for use according to claim 3, wherein the NK₁ inhibitor is fosaprepitant dimeglumine.

6. A NK₁ inhibitor as defined in any one of the previous claims, for use in treating:
breast cancer, wherein at least 1% of the cells in a breast cancer tumour to be treated do not express estrogen receptor and do not express progesterone receptor, and at least 10% of the cells in a breast cancer tumour to be treated do not express HER2, wherein expression of estrogen receptor, progesterone receptor and HER2 is measured by immunohistochemistry.

7. The pharmaceutical composition for use according to any one of claims 1 to 5 or the NK₁ inhibitor for use according to claim 6, wherein: (i) for a tumour that does not express estrogen receptor, less than 1% of cells in the tumour demonstrate nuclear positive staining for the estrogen receptor; (ii) for a tumour that does not express progesterone receptor less than 1% of cells in the tumour demonstrate nuclear positive staining for the progesterone receptor; and (iii) for a tumour that does not express HER2, HER2 staining is not observed in the tumour, or membrane staining of HER2 is incomplete, faint and/or barely perceptible within ≤ 10% of the tumour cells.

8. The pharmaceutical composition for use according to any one of claims 1 to 5 or 7 or the NK₁ inhibitor for use according to claim 6 or 7, wherein the NK₁ inhibitor is administered or used at a dose between 5 and 100 mg/kg/day, preferably between 5 and 50 mg/kg/day.

9. The pharmaceutical composition for use or the NK₁ inhibitor for use according to claim 7 or 8, wherein the NK₁ inhibitor is administered or used at a dose between 5 and 35 mg/kg/day, preferably between 5 and 25 mg/kg/day, more preferably between 5 and 15 mg/kg/day, most preferably between 5 and 10 mg/kg/day.

10. The pharmaceutical composition for use or the NK₁ inhibitor for use according to claim 7 or 8, wherein the NK₁ inhibitor is administered or used at a dose between 5 and 35 mg/kg/day, preferably between 10 and 35 mg/kg/day, more preferably between 15 and 35 mg/kg/day, most preferably between 25 and 35 mg/kg/day.
